# FASCICULE DE BREVET EUROPEEN

(11) **EP 3 071 213 B1**
(45) Date de publication et mention de la délivrance du brevet: **02.12.2020**
(21) Numéro de dépôt: 14801999.5
(22) Date de dépôt: 18.11.2014
(51) Int. Cl.: A61K 36/05, A61P 31/04

(54) **EXTRAIT D'ALGUES POUR SON UTILISATION EN TANT QU'AGENT ANTI-BACTÉRIEN**
ALGENEXTRAKT ZUR VERWENDUNG ALS ANTIBAKTERIELLES MITTEL
EXTRACT OF ALGAE FOR USE AS AN ANTIBACTERIAL AGENT

(30) Priorité: 18.11.2013 FR 1361297
(43) Date de publication de la demande: 28.09.2016
(73) Titulaire: Amadeite, 56580 Brehan (FR)
(72) Inventeur: DEMAIS, Hervé, F-56700 Merlevenez (FR); NYVALL COLLÈN, Pi, F-29680 Roscoff (FR); LE GOFF, Matthieu, F-56800 Ploermel (FR); LE CHEVILLER, Isabelle, F-56580 Bréhan (FR)
(74) Mandataire: Lavoix
(86) Numéro de dépôt international: PCT/EP2014/074944
(87) Numéro de publication internationale: WO 2015/071502

(56) Documents cités:
- JP-A- H1 180 003
- US-A1- 2003 181 416
- US-A1- 2011 104 189
- US-A1- 2013 065 851
- DATABASE WPI Week 200911 Thomson Scientific, London, GB; AN 2009-B40340 XP002734753, & KR 2008 0069735 A (AMBIO CO LTD) 29 juillet 2008 (2008-07-29)
- VERA JEANNETTE ET AL: "Seaweed Polysaccharides and Derived Oligosaccharides Stimulate Defense Responses and Protection Against Pathogens in Plants", MARINE DRUGS, vol. 9, no. 12, décembre 2011 (2011-12), pages 2514-2525, XP002725876,
- PIERRE GUILLAUME ET AL: "Antibacterial Activity of a Sulfated Galactan Extracted from the Marine Alga Chaetomorpha aerea Against Staphylococcus aureus", BIOTECHNOLOGY AND BIOPROCESS ENGINEERING, vol. 16, no. 5, octobre 2011 (2011-10), pages 937-945, XP002725877,
- DATABASE WPI Week 200962 Thomson Scientific, London, GB; AN 2009-N18962 XP002725878, & PT 103 983 A1 (NECTON CIA PORTUGUESA CULTURAS MARINHAS SA) 31 août 2009 (2009-08-31)
- HUIMIN QI ET AL: "Antioxidant activity of different molecular weight sulfated polysaccharides from Ulva pertusa Kjellm (Chlorophyta)", JOURNAL OF APPLIED PHYCOLOGY, KLUWER ACADEMIC PUBLISHERS, DO, vol. 17, no. 6, 1 décembre 2005 (2005-12-01), pages 527-534, XP019247834, ISSN: 1573-5176
- MARC LAHAYE ET AL: JOURNAL OF APPLIED PHYCOLOGY, vol. 11, no. 1, 1 janvier 1999 (1999-01-01), pages 1-7, XP055068568, ISSN: 0921-8971, DOI: 10.1023/A:1008063600071

## Description

La présente invention concerne un extrait d'algues de l'ordre des ulvales, en particulier un extrait d'algues vertes de type *Ulva*, pour son utilisation dans la prévention et/ou le traitement d'une infection bactérienne.

L'utilisation abusive et excessive d'agents antimicrobiens comme promoteurs de croissance dans l'alimentation des animaux d'élevage pendant des décennies, a créé une pression sélective continue et le développement de souches résistantes, résultant en une interdiction complète de l'utilisation de promoteurs de croissance antimicrobiens dans l'Union européenne (règlement (CE) n°1831/2003). Des stratégies prophylactiques alternatives capables de limiter l'infection de ces animaux sont donc nécessaires.

*Staphylococcus aureus* est l'espèce la plus pathogène du genre *Staphylococcus.* Cette bactérie est depuis longtemps reconnue comme étant un agent pathogène responsable d'un large éventail de maladies et d'infections, allant des infections cutanées mineures aux infections de plaies mortelles graves.

Bien que cette bactérie colonise fréquemment le nez et la peau des êtres humains en bonne santé, ce qui provoque alors souvent uniquement des infections mineures (par exemple des boutons), elle peut aussi causer des infections systémiques.

Les infections cutanées sont toutefois les plus fréquentes (Waldvogel FA. Staphylococcus aureus. In: Mandell I, Gerald L, Dolin R, eds. Mandell, Douglas and Bennett's Principles and Practice of Infectious Diseases. New York: Churchill Linvingstone; 1995. p. 1754-77) et sont représentées par des infections locales comme les folliculites, les furoncles, l'impétigo (Williams RE, MacKie RM. The staphylococci. Importance of their control in the management of skin disease. Dermatol Clin 1993 ; 11 : 201-6), les hydradénites et les mastites. Certaines formes de staphylococcies cutanées prennent une apparence de maladie systémique comme le syndrome des enfants ébouillantés ou le syndrome de choc toxique staphylococcique (TSS). Les staphylocoques sont également capables de coloniser et d'infecter des lésions dermatologiques comme la dermatite atopique (Higaki S, Morohashi M, Yamagishi T, Hasegawa Y. Comparative study of staphylococci from the skin of atopic dermatitis patients and from healthy subjects. Int J Dermatol 1999 ; 38 : 265-9).

L'incidence mondiale croissante des infections à staphylocoques est fortement liée à l'utilisation accrue de dispositifs chirurgicaux ainsi qu'au nombre croissant de patients immunodéprimés. Par ailleurs, l'utilisation accrue des antibiotiques a conduit à l'émergence de souches de S. *aureus* résistantes à la méthicilline (SARM) (Selvey et al., 2000, Infect. Control. Hosp. Epidemiol. 21:645-8; Peacock et al., 1980, Ann. Intern. Med. 93:526-32).

D'autres bactéries causant chez l'homme ou l'animal des infections et pathologies diverses nécessitent également la recherche de traitements alternatifs aux antibiotiques classiques.

L'accroissement de la résistance de ces bactéries aux agents antimicrobiens nécessite de trouver des solutions pour le traitement des infections résistantes aux antibiotiques classiques et pour lutter contre le développement de nouvelles souches résistantes.

Les espèces d'ulves (Ulvales, Chlorophyta) sont des algues abondantes trouvées dans la zone intertidale ou estran. Elles colonisent les substrats durs, ancrés par un disque de fixation, mais certaines espèces peuvent également donner lieu à des formes vivantes libres, à la dérive. Les ulves sont des algues à croissance rapide et opportunistes quant à l'espace et l'absorption des nutriments. Leur croissance dans la colonne d'eau est particulièrement observée dans les eaux côtières eutrophisées et dans les lagunes où *Ulva* sp. prolifère sous la forme de "marées vertes" (Fletcher, 1996). Il en résulte souvent une production de masse et des échouages massifs produisant des gaz nocifs lors de leur accumulation (Morand et Briand, 1996). Jusqu'à présent, cette biomasse a possédé une très faible valeur ajoutée et les moyens de l'utiliser en dehors du compost (Mazé et al. 1993, Cuomo et al. 1995), de la production de méthane (Briand et Morand, 1997), de la consommation alimentaire humaine (Pérez, 1997) ou comme base de papier (Nicolucci et Monegato 1993) pourraient permettre de profiter de leurs propriétés spécifiques.

Le brevet KR 2008 0069735 mentionne des extraits d'*Ulva lactula* ayant des propriétés antibactériennes, toutefois sans préciser la nature du principe actif.

Ainsi, la présente invention concerne un extrait d'algues de l'ordre des ulvales, en particulier un extrait d'algues vertes de type *Ulva*, comprenant des polysaccharides polyanioniques sulfatés et non sulfatés dont la taille est inférieure à 50 kDa, pour son utilisation dans la prévention et/ou le traitement d'une infection bactérienne.

En particulier, la présente invention concerne un extrait d'algues pour son utilisation telle que définie ci-dessus, dans laquelle les polysaccharides comprennent du mannose et/ou de l'arabinose, préférentiellement du mannose. Plus particulièrement encore, lesdits polysaccharides comprennent au moins 0.005% de mannose et/ou au moins 0.005% d'arabinose, en poids par rapport au poids de la matière sèche totale de l'extrait d'algues, préférentiellement au moins 0.005% de mannose. Encore plus particulièrement, lesdits polysaccharides comprennent du mannose en une quantité allant de 0.005 à 0.5%, par exemple de 0.005 à 0.20% ou de 0.15 à 0.5% et/ou de l'arabinose en une quantité allant de 0.005 à 0.5%, en poids par rapport au poids de la matière sèche totale de l'extrait d'algues, préférentiellement du mannose en une quantité allant de 0.005 à 0.5%, par exemple de 0.005 à 0.20% ou de 0.15 à 0.5% en poids par rapport au poids de la matière sèche totale de l'extrait d'algues. La présente invention concerne plus particulièrement encore un extrait d'algues pour son utilisation tel que définie ci-dessus, dans laquelle lesdits polysaccharides comprennent en outre:
- du galactose; et/ou
- du glucose; et/ou
- du rhamnose; et/ou
- du xylose; et/ou
- de l'acide glucuronique.

Plus particulièrement, lesdits polysaccharides comprennent :
- de 0.05 à 0.5% de galactose; et/ou
- de 0.005 à 0.5% de glucose, en particulier de 0.005 à 0.05% ou de 0.05 à 0.5% ; et/oude 2 à 15 % de rhamnose; et/ou
- de 0.1 à 1% de xylose; et/ou
- de 1 à 7% d'acide glucuronique;
en poids par rapport au poids de la matière sèche totale de l'extrait d'algues.

L'extrait ci-dessus mentionné est susceptible d'être obtenu par un procédé de préparation dans lequel:
a) les algues sont lavées et dessablées ;
b) lesdites algues sont broyées ;
c) la phase solide du broyat est séparée de sa phase liquide ;
d) ladite phase liquide est clarifiée ;
e) le jus obtenu à l'étape d) est ultrafiltré sur une membrane de moins de 50 kDa; et
f) le jus de filtration obtenu à l'étape e) est concentré puis séché ;
pour son utilisation dans la prévention et/ou le traitement d'une infection bactérienne.

Par « infection bactérienne », on entend la signification habituellement apportée à ce terme et bien connue de l'homme du métier, en particulier une maladie infectieuse causée par une bactérie. Les infections bactériennes sont très nombreuses en pathologie humaine et vétérinaire. Les germes arrivent dans l'organisme après une contamination directe ou bien indirecte. Ils peuvent être présents dans l'alimentation ou sur les objets souillés et contaminés. Des vecteurs animés ou bien immobiles peuvent également transmettre les bactéries.

En particulier, dans le cadre de la présente invention, l'infection bactérienne est une infection par une bactérie à Gram positif ou Gram négatif. Les bactéries à Gram positif ou négatif sont mises en évidence par une technique de coloration appelée coloration de Gram. Les bactéries à Gram négatif apparaissent alors roses au microscope tandis que les bactéries à Gram positif apparaissent en violet.

Comme bactéries à Gram positif, on peut par exemple citer :
- Genre *Staphylococcus* ;
- Genre *Micrococcus* ;
- Genre *Lactococcus* ;
- Genre *Lactobacillus* ;
- Genre *Clostridium* ;
- Genre *Bacillus* ;
- Genre *Streptococcus* ;
- Genre *Erysipelothrix* ;
- Genre *Corynebacterium* ;
- Genre *Actinomyces* ;
- Genre *Enterococcus* ; et
- Genre *Listeria.*

On peut citer en particulier les bactéries à Gram positif suivantes : *Staphylococcus aureus, Listeria monocytogenes, Staphyloccocus sciuri, Staphyloccocus simulans, Staphyloccocus xylosus, Staphylococcus chromogenes, Staphylococcus epidermidis, Staphylococcus haemolyticus, Streptococcus cristatus, Streptococcus suis II, Streptococcus dysgalactiae spp dysgalactiae, Streptococcus dysgalactiae, Streptococcus equinus, Streptococcus uberis, Erysipelothrix, Enterococcus cecorum, Enterococcus faecalis, Enterococcus faecium, Lactococcus garvieae, Corynebacterium bovis, Actinomyces pyogenes.*

Comme bactéries à Gram négatif, on peut par exemple citer :
- Famille des *Alcaligenaceae* :
   ∘ Genre *Bordetella*
- Famille des *Enterobacteriaceae :*
   ∘ Genre *Salmonella*
   ∘ Genre *Escherichia coli*
   ∘ Genre *Serratia*
   ∘ Genre *Klebsiella*
   ∘ Genre *Yersinia* exemple: *Yersinia pestis* (responsable de la peste)
- Famille des *Pasteurellaceae* :
   ∘ Genre *Pasteurella*
- Espèce des *Vibrionaceae* :
   ∘ Genre *Vibrio cholerae* (responsable du choléra)
- Espèce des *Pseudomonadaceae :*
   ∘ Genre *Pseudomonas*
- Espèce des *Neisseriaceae :*
   ∘ Genre *Neisseria meningitidis* (responsable de la méningite bactérienne)
- Espèce des *Rhizobiaceae :*
   ∘ Genre *Agrobacterium tumefaciens* (responsable de la galle du collet)
   ∘ Genre *Agrobacterium rhizogenes*
- Genre *Mannheimia*

On peut citer en particulier les bactéries à Gram négatif suivantes : *Escherichia coli, Sallmonella typhimurium, Sallmonella Hadar, Sallmonella Infantis*, *Sallmonella Virchow, Sallmonella Enteritidis, Sallmonella Typhimurium, Klebsiella pneumoniae, Citrobacter braaki, Citrobacter gillenii, Enterobacter cloacae, Proteus mirabilis, Serratia marcescens, Pasteurella m. subsp septica, Pasteurella multocida subsp gallicida ST, Mannheimia haemolytica, Pasteurella multocida, Pasteurella multocida subsp multocida ST, Mannheimia, Bordetella bronchiseptica, Serratia marcescens.*

Plus particulièrement, dans le cadre de la présente invention, l'infection bactérienne est une infection par une bactérie à Gram positif choisie parmi *Staphylococcus aureus* et *Listeria monocytogenes* ou à Gram négatif choisie parmi *Escherichia coli* et *Sallmonella typhimurium.*

Plus particulièrement encore, dans le cadre de la présente invention, l'infection bactérienne est une infection bactérienne causée par *Staphyloccocus aureus,* notamment une infection bactérienne cutanée.

Ainsi, l'extrait d'algues pour son utilisation selon la présente invention sera notamment utile pour prévenir et/ou traiter toute infection causée par l'une des bactéries précédemment mentionnées, comme par exemple une métrite ou une mammite, en particulier une infection causée par *Staphylococcus aureus,* plus particulièrement encore pour prévenir et/ou traiter une infection bactérienne cutanée causée par *Staphyloccocus aureus,* comme par exemple des infections locales telles que les folliculites, les furoncles, l'impétigo, les hydradénites et les mastites, certaines formes de staphylococcies cutanées prenant une apparence de maladie systémique comme le syndrome des enfants ébouillantés ou le syndrome de choc toxique staphylococcique (TSS).

En particulier également, dans le cadre de la présente invention, l'infection bactérienne est causée par *Pasteurella m. subsp septica*, *Pasteurella multocida subsp gallicida ST, Mannheimia haemolytica, Pasteurella multocida, Pasteurella multocida subsp multocida ST, Mannheimia, Erysipelothrix, Staphylococcus aureus volaille, Staphylococcus aureus bovins, Staphylococcus aureus poulet, Streptococcus suis II*, *Enterococcus cecorum, Streptococcus dysgalactiae ssp dysgalactiae, Streptococcus dysgalactiae*, *Bordetella bronchiseptica, Corynebacterium bovis, Actinomyces pyogenes, Staphylococcus chromogenes, Streptocoque uberis, Listeria monocytogenes* ou *Serratia marcescens* ; en particulier par *Pasteurella m. subsp septica, Pasteurella multocida subsp gallicida ST*, *Mannheimia haemolytica, Pasteurella multocida, Pasteurella multocida subsp multocida ST, Mannheimia, Erysipelothrix, Staphylococcus aureus volaille, Staphylococcus aureus bovins, Staphylococcus aureus poule, Streptococcus suis II*, *Enterococcus cecorum, Streptococcus dysgalactiae ssp dysgalactiae, Streptococcus dysgalactiae, Bordetella bronchiseptica, Corynebacterium bovis, Actinomyces pyogenes, Staphylococcus chromogenes* ; et encore plus particulièrement par *Pasteurella m. subsp septica, Pasteurella multocida subsp gallicida ST, Mannheimia haemolytica*, *Pasteurella multocida, Pasteurella multocida subsp multocida ST*, *Mannheimia, Erysipelothrix, Staphylococcus aureus volaille, Staphylococcus aureus bovins, Staphylococcus aureus poulet* ou *Streptococcus suis II.*

L'extrait d'algues pour son utilisation selon la présente invention sera donc notamment utile pour prévenir et/ou traiter toute infection causée par l'une des bactéries précédemment mentionnées, comme par exemple chez l'animal : la colibacillose, la septicémie, l'infection viscérale, la lésion viscérale, la salmonellose, la diarrhée, l'hyperthermie, la dermite exsudative, la furonculose, la mammite, l'abcès, l'arthrite, l'ostéomyélite, les troubles locomoteurs, la septicémie hémorragique, la fièvre des transports, la pneumonie, l'endocardite, la méningite, la septicémie aigue, la polyarthrite, la lésion cutanée érythémateuse, la pneumonie, l'omphalite, l'entérite du lapin (diarrhée hémorragique) ainsi que pour prévenir le portage de ces souche et leur retransmission à l'homme via la consommation des produits animales infectés; chez l'homme : la diarrhée, les infections alimentaires, les intoxications alimentaires, les maladies gastro-intestinales, les furoncles, les folliculites, les panaris, l'impétigo, l'abcès mammaire chez les femmes qui allaitent, les infections des muqueuses, en particulier celles pouvant atteindre les yeux (conjonctivites), les oreilles (otites), la sphère génitale (endométrite, salpingite) ou les voies respiratoires (pneumonies, pleurésies), le choc toxique staphylococcique, la scarlatine staphylococcique, le syndrome d'exfoliation généralisée, l'impétigo bulbeux, une infection nosocomiale respiratoire ; en particulier chez l'animal : la septicémie, la dermite exsudative, la furonculose, la mammite, l'abcès, l'arthrite, la septicémie hémorragique, la fièvre des transports, la pneumonie, l'endocardite, la méningite, la septicémie aigue, la lésion cutanée érythémateuse, et en particulier chez l'homme : les furoncles, les folliculites, les panaris, l'impétigo, l'abcès mammaire chez les femmes qui allaitent, les infections des muqueuses, en particulier celles pouvant atteindre les yeux (conjonctivites), les oreilles (otites), la sphère génitale (endométrite, salpingite) ou les voies respiratoires (pneumonies, pleurésies), le choc toxique staphylococcique, la scarlatine staphylococcique, le syndrome d'exfoliation généralisée, l'impétigo bulbeux. Ainsi, l'extrait d'algues pour son utilisation selon la présente invention sera notamment utile pour prévenir et/ou traiter toute infection causée par l'une des bactéries susmentionnées, en particulier par *Pasteurella m. subsp septica, Pasteurella multocida subsp gallicida ST*, *Mannheimia haemolytica, Pasteurella multocida*, *Pasteurella multocida subsp multocida ST, Mannheimia, Erysipelothrix, Staphylococcus aureus volaille, Staphylococcus aureus bovins, Staphylococcus aureus poulet* ou *Streptococcus suis II.*

Un extrait d'algues pour son utilisation selon la présente invention est notamment décrit dans la demande de brevet FR1261909.

Tel qu'indiqué ci-dessus, l'extrait d'algues pour son utilisation selon la présente invention est un extrait d'algues de l'ordre des ulvales, en particulier un extrait d'algues vertes de type *Ulva*, comprenant des polysaccharides polyanioniques sulfatés et non sulfatés dont la taille est inférieure à 50 kDa. Plus particulièrement, l'extrait d'algues pour son utilisation selon la présente invention comprend des polysaccharides polyanioniques sulfatés et non sulfatés dont la taille est inférieure à 40, 30, 20 ou 15 kDa. Plus particulièrement encore, les polysaccharides polyanioniques sulfatés et non sulfatés de l'extrait d'algues pour son utilisation selon l'invention ont une taille inférieure ou égale à 15 kDa.

Selon un mode réalisation de l'invention, l'extrait d'algues tel que décrit dans la cadre de la présente invention comprend des polysaccharides polyanioniques sulfatés et non sulfatés dont la taille est inférieure à 50 kDa, à l'exclusion de polysaccharides polyanioniques sulfatés et non sulfatés dont la taille est supérieure ou égale à 50 kDa.

Selon un autre mode réalisation de l'invention, l'extrait d'algues tel que décrit dans le cadre de la présente invention comprend des polysaccharides polyanioniques sulfatés et non sulfatés dont la taille est inférieure ou égale à 15 kDa, à l'exclusion de polysaccharides polyanioniques sulfatés et non sulfatés dont la taille est supérieure à 15 kDa.

Un dalton (Da) est une unité massique définie comme étant égal à un douzième de la masse d'un atome de carbone 12, masse qui s'avérera ensuite estimée à partir d'un mélange de plusieurs isotopes (principalement carbone 12 et carbone 13, possédant respectivement 6 et 7 neutrons en plus des 6 protons de tout atome de carbone). Un dalton est, avec une assez bonne précision, la masse d'un atome d'hydrogène, la valeur exacte étant 1,00794 uma (unité de masse atomique). Le kilodalton (kDa) est égal à 1000 Da.

Dans le cadre de la présente invention, les masses mentionnées en kDa sont déterminées par toute méthode usuellement employée par l'homme du métier, en particulier les masses des polysaccharides polyanioniques sulfatés et non sulfatés des extraits d'algues selon la présente invention pourront être discriminées par ultrafiltration sur des membranes laissant uniquement filtrer des molécules de tailles prédéterminées.

En particulier, et tel que précédemment mentionné, les polysaccharides de l'extrait d'algues pour son utilisation selon la présente invention comprennent du mannose et/ou de l'arabinose, de préférence du mannose. Plus particulièrement, lesdits polysaccharides comprennent au moins 0.005% de mannose et/ou au moins 0.005% d'arabinose, en poids par rapport au poids de la matière sèche totale de l'extrait d'algues, notamment au moins au 0.01 % de mannose et/ou au moins 0.01 % d'arabinose. Encore plus particulièrement, lesdits polysaccharides comprennent du mannose en une quantité allant de 0.01 à 0.50%, par exemple de 0.01 à 0.20% ou de 0.20 à 0.5% et/ou de l'arabinose en une quantité allant de 0.01 à 0.5%, en poids par rapport au poids de la matière sèche totale de l'extrait d'algues, notamment du mannose en une quantité allant de 0.03 à 0.45%, par exemple de 0.03 à 0.15% ou de 0.15 à 0.45% et/ou de l'arabinose en une quantité allant de 0.01 à 0.2%.

Préférentiellement, lesdits polysaccharides comprennent du mannose en une quantité allant de 0.01 à 0.50%, par exemple de 0.01 à 0.20% ou de 0.20 à 0.5%, notamment du mannose en une quantité allant de 0.03 à 0.45%, par exemple de 0.03 à 0.15% ou de 0.15 à 0.45%.

De façon particulière, et tel que mentionné précédemment, lesdits polysaccharides comprennent en outre:
- du galactose; et/ou
- du glucose; et/ou
- du rhamnose; et/ou
- du xylose; et/ou
- de l'acide glucuronique.

Plus particulièrement encore, lesdits polysaccharides comprennent :
- de 0.05 à 0.5% de galactose, notamment de 0.1 à 0.4%; et/ou
- de 0.005 à 0.5% de glucose, en particulier de 0.005 à 0.05%, notamment de 0.01 à 0.03%, ou de 0.05 à 0.5% ; et/ou
- de 2 à 15 % de rhamnose, notamment de 5 à 10%; et/ou
- de 0.1 à 1% de xylose, notamment de 0.3 à 0.7%; et/ou
- de 1 à 7% d'acide glucuronique, notamment de 1 à 5%;
en poids par rapport au poids de la matière sèche totale de l'extrait d'algues.

Ainsi, on peut par exemple citer un extrait d'algues pour son utilisation selon l'invention comprenant :
- du mannose ; et/ou
- de l'arabinose ; et/ou
- du galactose; et/ou
- du glucose; et/ou
- du rhamnose; et/ou
- du xylose; et/ou
- de l'acide glucuronique.
On peut plus particulièrement citer par exemple un extrait d'algues pour son utilisation selon l'invention comprenant :
- de 0.01 à 0.50% de mannose, par exemple de 0.01 à 0.20%, notamment de 0.03 à 0.15% ou de 0.20 à 0.5%; et/ou
- de 0.01 à 0.5% d'arabinose, notamment de 0.01 à 0.2% ; et/ou
- de 0.05 à 0.5% de galactose, notamment de 0.1 à 0.4%; et/ou
- de 0.005 à 0.5% de glucose, en particulier de 0.005 à 0.05%, notamment de 0.01 à 0.03%, ou de 0.05 à 0.5%et/ou
- de 2 à 15 % de rhamnose, notamment de 5 à 10%; et/ou
- de 0.1 à 1% de xylose, notamment de 0.3 à 0.7%; et/ou
- de 1 à 7% d'acide glucuronique, notamment de 1 à 5%;
en poids par rapport au poids de la matière sèche totale de l'extrait d'algues.
On peut encore plus particulièrement citer par exemple un extrait d'algues pour son utilisation selon l'invention comprenant :
- 0.09% de mannose; et/ou
- 0.1% d'arabinose ; et/ou
- 0.3% de galactose; et/ou
- 0.02% de glucose ; et/ou
- 8.1% de rhamnose; et/ou
- 0.5% de xylose; et/ou
- 2.6% d'acide glucuronique;
en poids par rapport au poids de la matière sèche totale de l'extrait d'algues.
On peut également citer par exemple un extrait d'algues pour son utilisation selon l'invention comprenant :
- 0.3% de mannose; et/ou
- 0.2% de galactose; et/ou
- 0.4% de glucose ; et/ou
- 7.9% de rhamnose; et/ou
- 0.5% de xylose; et/ou
- 4.9% d'acide glucuronique;
en poids par rapport au poids de la matière sèche totale de l'extrait d'algues.

Tel qu'indiqué ci-dessus, l'extrait d'algues pour son utilisation selon la présente l'invention est également un extrait d'algues de l'ordre des ulvales, en particulier un extrait d'algues vertes de type *Ulva*, susceptible d'être obtenu par un procédé de préparation dans lequel:
a) les algues sont lavées et dessablées ;
b) lesdites algues sont broyées ;
c) la phase solide du broyat est séparée de sa phase liquide ;
d) ladite phase liquide est clarifiée ;
e) le jus obtenu à l'étape d) est ultrafiltré sur une membrane de moins de 50 kDa; et le jus de filtration obtenu à l'étape e) est concentré puis séché.

Selon un mode de réalisation de l'invention, l'extrait d'algues comprend :
- de 10 à 50 % de carbone ;
- de 1 à 10% d'hydrogène ;
- de 1 à 5 % d'azote ;
- de 20 à 50 % d'oxygène ; et
- de 1 à 15 % de soufre ;
en pourcentage massique de la matière sèche totale de l'extrait d'algues.

Plus particulièrement encore, l'extrait d'algues comprend :
- de 15 à 30 % de carbone ;
- de 3 à 6% d'hydrogène ;
- de 1 à 3 % d'azote ;
- de 25 à 40 % d'oxygène ; et
- de 2,5 à 10 % de soufre ;
en pourcentage massique de la matière sèche totale de l'extrait d'algues.

Selon un autre mode de réalisation de l'invention, l'extrait d'algues comprend :
- de 10 à 50 % de carbone ;
- de 1 à 10% d'hydrogène ;
- de 0,5 à 5 % d'azote ;
- de 20 à 60 % d'oxygène ; et
- de 1 à 15 % de soufre ;
en pourcentage massique de la matière sèche totale de l'extrait d'algues.

Les autres éléments chimiques présents dans la matière sèche de l'extrait sont notamment représentés par les minéraux (Ca, K, Na, Mg, Al, Cl, I, P, Fe, etc).

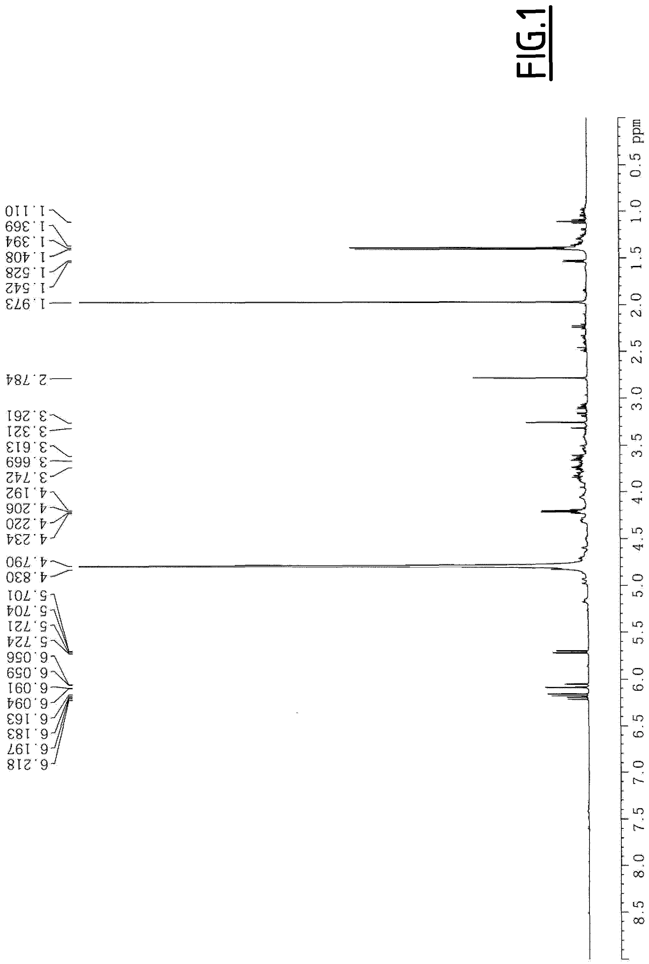

Plus particulièrement, l'extrait d'algues pour son utilisation selon l'invention est caractérisé par le spectre RMN ¹H présenté en Figure 1.

Ce spectre RMN ¹H a été enregistré à 298 K sur un spectromètre Bruker Avance 500 équipé d'une sonde cryogénique inverse 5 mm ¹H/¹³C/¹⁵N TCl. Avant l'analyse, les échantillons ont été dissous dans 99,97% d'atome de D₂O. Les déplacements chimiques sont exprimés en ppm par rapport à un étalon externe (acide triméthylsilylpropionique). Aucune suppression du signal HOD n'a été réalisée.

Selon un mode de réalisation de la présente invention, l'extrait d'algues comprenant en particulier des polysaccharides polyanioniques sulfatés et non sulfatés dont la taille est inférieure à 50 kDa est susceptible d'être obtenu par un procédé de préparation dans lequel:
a) les algues sont lavées et dessablées ;
b) lesdites algues sont broyées ;
c) la phase solide du broyat est séparée de sa phase liquide ;
d) ladite phase liquide est clarifiée ;
e) le jus obtenu à l'étape d) est ultrafiltré sur une membrane de moins de 50 kDa; et
f) le jus de filtration obtenu à l'étape e) est concentré puis séché.

En particulier, pour la mise en œuvre du procédé tel qu'indiqué dans le cadre de la présente invention, dans l'étape a) de celui-ci, les algues sont lavées à l'eau douce.

Elles peuvent être dessablées par tout moyen mis à disposition de l'homme du métier.

Lesdites algues sont ensuite broyées, notamment au moyen d'un broyeur, comme par exemple un affineur ou un cutteur.

Par la suite, la phase solide du broyat, le marc, est séparée de sa phase liquide, le jus, par pressage du broyat, par exemple à l'aide d'une presse à bande ou à plateaux, ou par centrifugation.

Par « jus », on entend le jus cytoplasmique qui inclue la structure pariétale de la double structure des cellules des algues.

La phase liquide obtenue est ensuite clarifiée, par exemple avec un clarificateur à assiettes, ou par centrifugation, décantation ou filtration (par exemple à poche ou à plaque).

Le jus obtenu est ensuite ultrafiltré.

Selon un mode de réalisation pour la mise en œuvre du procédé tel qu'indiqué dans le cadre de la présente invention, l'ultrafiltration est réalisée sur une membrane de de moins de 50 KDa, en particulier sur une membrane de 40, 30, 20 ou 15 kDa. Plus particulièrement, la membrane sera une membrane de 15 kDa ou moins.

Cette membrane peut être par exemple une membrane de céramique ou une membrane organique. Plus particulièrement, la membrane sera une membrane de céramique.

Le jus de filtration obtenu peut ensuite être concentré, par exemple par osmose inverse, évaporation ou précipitation, puis séché par exemple par lyophilisation ou atomisation.

Optionnellement, l'extrait obtenu pourra ensuite être broyé de nouveau, afin d'obtenir une poudre homogène en terme de granulométrie.

Selon un de ces aspects, le procédé se déroule en partie à température ambiante. Par température ambiante, on entend une température comprise entre 5 et 25°C.

Selon un autre de ces aspects, le procédé se déroule en partie à une température comprise entre 4 et 10°C, ceci afin d'éviter les développements microbiens.

Selon un mode de réalisation pour la mise en œuvre du procédé tel qu'indiqué dans le cadre de la présente invention, l'extrait d'algues obtenu à l'étape f) du procédé susmentionné est purifié, par exemple par ultrafiltration, en particulier sur une cassette d'ultrafiltration, notamment pour éliminer la partie minérale.

Selon un autre de ces aspects, l'extrait d'algues pour son utilisation selon l'invention est obtenu par le procédé tel que précédemment décrit.

Ce procédé diffère de la plupart des procédés décrits dans l'art antérieur du fait de l'absence d'une étape impliquant une précipitation de l'extrait d'algues. Il se distingue également des précédents procédés d'extraction de par l'absence d'utilisation de solvants, en particulier organiques, ce qui représente un atout majeur du point de vue écologique.Par « algues vertes de type *Ulva* » on entend les algues vertes regroupées dans le genre *Ulva*, de la famille des Ulvaceae, de l'ordre des ulvales. On peut notamment citer les espèces et sous-espèces suivantes : *Ulva acanthophora, Ulva anandii, Ulva angusta, Ulva arasakii, Ulva armoricana, Ulva atroviridis, Ulva attenuata, Ulva beytensis, Ulva bifrons, Ulva brevistipitata, Ulva bulbosa, Ulva burmanica, Ulva byssoides, Ulva californica, Ulva chaetomorphoides, Ulva clathrata, Ulva coccinea, Ulva compressa, Ulva conglobata, Ulva cornucopiae, Ulva cornuta, Ulva covelongensis, Ulva crassa, Ulva crassimembrana, Ulva curvata, Ulva dactylifera, Ulva denticulata, Ulva elegans, Ulva elminthoides, Ulva enteromorpha, Ulva erecta, Ulva expansa, Ulva fasciata, Ulva fenestrata, Ulva flexuosa, Ulva gelatinosa, Ulva geminoidea, Ulva gigantea, Ulva grandis, Ulva hendayensis, Ulva hookeriana, Ulva hopkirkii, Ulva indica, Ulva intestinalis, Ulva intestinaloides, Ulva intricata, Ulva intybacea, Ulva javanica, Ulva kylinii, Ulva lactuca, Ulva lactucaefolia, Ulva laetevirens, Ulva laingii, Ulva linearis, Ulva lingulata, Ulva linkiana, Ulva linza, Ulva lippii, Ulva litoralis, Ulva littorea, Ulva lobata, Ulva lubrica, Ulva marginata, Ulva micrococca, Ulva myriotrema, Ulva neapolitana, Ulva nematoidea, Ulva ohnoi, Ulva olivacea, Ulva olivaceum, Ulva pacifica, Ulva papenfussii, Ulva paradoxa, Ulva parva, Ulva parvula, Ulva patengensis, Ulva percursa, Ulva pertusa, Ulva phyllosa, Ulva popenguinensis, Ulva porrifolia, Ulva procera, Ulva profunda, Ulva prolifera, Ulva pseudocurvata, Ulva pseudolinza, Ulva pulchra, Ulva purpurascens, Ulva quilonensis, Ulva radiata, Ulva ralfsii, Ulva ranunculata, Ulva reticulata, Ulva rhacodes, Ulva rigida, Ulva rotundata, Ulva rubens, Ulva saifullahii, Ulva scagelii, Ulva scandinavica, Ulva sericea, Ulva serrata, Ulva simplex, Ulva sorensenii, Ulva spinulosa, Ulva stenophylla, Ulva stipitata, Ulva sublittoralis, Ulva subulata, Ulva taeniata, Ulva tenera, Ulva tetragona, Ulva torta, Ulva tuberosa, Ulva umbilicata, Ulva uncialis, Ulva uncinata, Ulva usneoides, Ulva utricularis, Ulva utriculosa, Ulva uvoides, Ulva ventricosa.*

Ainsi, un extrait d'algues pour son utilisation selon la présente invention peut être utilisé aussi bien dans des applications vétérinaires, comme par exemple pour traiter les infections bactériennes cutanées causées l'une des bactéries susmentionnées, en particulier par *Staphyloccocus aureus,* que dans des applications destinées à l'être humain, par exemple par le biais d'un médicament ou d'une composition pharmaceutique, pour prévenir et/ou traiter les infections bactériennes, telles que celles mentionnées ci-dessus.

Ainsi la présente invention concerne également un extrait d'algues pour son utilisation telle que précédemment mentionnée, dans laquelle l'extrait d'algues est compris dans une composition, en particulier une composition pharmaceutique ou dans un médicament. Les excipients pharmaceutiquement acceptables utilisés pour la préparation d'un médicament ou d'une composition pharmaceutique comprenant un extrait d'algues pour son utilisation selon l'invention sont choisis selon la forme pharmaceutique et le mode d'administration souhaité, parmi les excipients habituels qui sont connus de l'homme du métier.

Dans la présente invention pour l'administration orale, sublinguale, sous-cutanée, intramusculaire, intra-veineuse, topique, locale, intratrachéale, intranasale, transdermique ou rectale, l'extrait d'algues tel que défini ci-dessus, peut être administré sous forme de doses unitaires, en mélange avec des excipients pharmaceutiques classiques, aux animaux et aux êtres humains, pour la prévention ou le traitement des infections bactériennes précitées.

Les formes d'administration appropriées comprennent les formes par voie orale telles que les comprimés, les gélules molles ou dures, les poudres, les granules et les solutions ou suspensions orales, les formes d'administration sublinguale, buccale, intratrachéale, intraoculaire, intranasale, par inhalation, les formes d'administration topique, parentérale telle que transdermique, sous cutanée, intramusculaire ou intraveineuse, les formes d'administration rectale et les implants. Pour l'application topique, on peut utiliser les extraits d'algues pour leur utilisation selon l'invention dans des crèmes, gels, pommades ou lotions.

Lorsqu'une composition solide sous forme de comprimés est préparée, l'ingrédient actif principal peut être mélangé avec un véhicule pharmaceutique, tel que la gélatine, l'amidon, le lactose, le stéarate de magnésium, le talc, la gomme arabique ou analogues.

Les comprimés peuvent également être enrobés avec du saccharose, un dérivé cellulosique, ou d'autres matières appropriées ou encore ils peuvent être traités de telle sorte qu'ils aient une activité prolongée ou retardée et qu'ils libèrent d'une façon continue une quantité prédéterminée de principe actif.

Une préparation sous forme de gélules peut par exemple être obtenue en mélangeant l'ingrédient actif avec un diluant et en versant le mélange obtenu dans des gélules molles ou dures.

Dans un mode de réalisation particulier, l'extrait d'algues, le médicament ou la composition pharmaceutique pour son utilisation selon la présente invention est destiné à une administration orale.

Les médicaments ou compositions pharmaceutiques comprenant un extrait d'algues pour son utilisation selon l'invention, peuvent aussi se présenter sous forme liquide, par exemple, sous forme de solutions, d'émulsions, de suspensions ou de sirops, et notamment sous une forme adaptée pour une administration orale ou intranasale, par exemple. Les supports liquides appropriés peuvent être, par exemple, de l'eau, des solvants organiques tels que le glycérol ou les glycols, de même que leurs mélanges, dans des proportions variées, dans l'eau.

Une préparation sous forme de sirop ou d'élixir ou pour l'administration sous forme de gouttes peut également contenir l'ingrédient actif conjointement avec un édulcorant, acalorique par exemple, du méthylparabène et du propylparabène comme antiseptique, ainsi qu'un agent donnant du goût et un colorant approprié.

Les poudres ou les granules dispersibles dans l'eau peuvent par exemple contenir l'ingrédient actif en mélange avec des agents de dispersion ou des agents mouillants, ou des agents de mise en suspension, comme la polyvinylpyrrolidone, de même qu'avec des édulcorants ou des correcteurs de goût. En général, la dose journalière de l'extrait d'algues pour son utilisation selon l'invention sera la dose efficace la plus faible de l'extrait d'algues capable de produire un effet thérapeutique.

Par « dose efficace », on désigne toute quantité d'une composition qui améliore un ou plusieurs des paramètres caractéristiques de la pathologie ou de l'infection à traiter.

Selon un des aspects de la présente invention, l'extrait d'algues est administré à une dose comprise entre 1 et 150 mg/kg, en particulier entre 10 et 120 mg/kg et plus particulièrement entre 20 et 100 mg/kg.

La présente invention, selon un autre de ces aspects, concerne également une méthode de réduction des probabilités d'occurrence ou de traitement des infections bactériennes telles que précédemment indiquées qui comprend l'administration, à un être humain ou un animal, d'une dose efficace d'un extrait d'algues tel que défini précédemment.

L'extrait d'algues administré dans le cadre de la méthode selon l'invention peut être compris dans un médicament ou une composition pharmaceutique tel que susmentionné.

Il peut être administré selon les modes d'administration susmentionnés.

Selon un mode de réalisation pour la mise en œuvre de la méthode selon l'invention, l'extrait d'algues selon l'invention est administré dans une composition pharmaceutique.

En particulier, la présente invention concerne également une méthode telle que susmentionnée qui comprend l'administration, à un être humain ou un animal, d'une dose efficace d'un extrait d'algues selon l'invention pendant une période de temps de 3 à 30 jours, en particulier de 10 à 20 jours ou de 3 à 10 jours, à une dose comprise entre 1 et 150 mg/kg, en particulier entre 10 et 120 mg/kg et plus particulièrement entre 20 et 100 mg/kg.

Selon un mode de réalisation pour la mise en œuvre de la méthode selon l'invention, à l'issu de la période de temps susmentionnée, l'administration de l'extrait d'algues peut être renouvelée pour une période équivalente.

Selon un de ces aspects, la présente invention concerne également une méthode telle que susmentionnée qui comprend :
a) la préparation d'un extrait d'algues selon l'invention par le procédé suivant :
   - lavage et dessablage des algues ;
   - broyage desdites algues;
   - séparation de la phase solide du broyat de sa phase liquide ;
   - clarification de ladite phase liquide;
   - ultrafiltration du jus obtenu à l'étape précédente sur une membrane de moins de 50 kDa; et
   - concentration puis séchage du jus de filtration obtenu à l'étape précédente ; suivie optionnellement d'une étape d'ultrafiltration, par exemple sur une cassette d'ultrafiltration ; et
b) l'administration, à un être humain ou un animal, d'une dose efficace d'un extrait d'algues selon l'invention, en particulier pendant une période de temps de 3 à 30 jours, en particulier de 10 à 20 jours ou de 3 à 10 jours, à une dose comprise entre 1 et 150 mg/kg, en particulier entre 10 et 120 mg/kg et plus particulièrement entre 20 et 100 mg/kg.

La présente invention sera illustrée plus en détail par les figures et exemples ci-dessous qui n'en limitent pas la portée.

### FIGURES

**Figure 1** : Spectre RMN ¹H d'un extrait d'algues (EA) tel que défini dans le cadre de la présente invention
**Figure 2** : Chromatogramme obtenu avec un extrait d'algues selon l'invention séparé sur deux colonnes shodex 802 et 803
**Figure 3** **:** Chromatogramme obtenu après l'analyse de dérivés triméthylsilylés de l'échantillon d'extrait d'algues selon l'invention par chromatographie en phase gazeuse. Avec Ara: Arabinose; Gal: Galactose; Glc: Glucose ; Xyl: Xylose; Man: Mannose; Rha: Rhamnose, GlcA : Acide Glucuronique

### EXEMPLES

### Exemple 1 : Préparation d'un extrait d'algues selon l'invention

Une tonne d'algues vertes de type *Ulva*, fraîches, brutes, sont lavées à l'eau douce et dessablées à l'aide d'une machine à laver les algues.

Sauf indications contraires, les étapes du procédé sont réalisées à température ambiante.

Les algues (1 tonne d'algues égouttées à 8% de matière sèche) sont ensuite broyées en fines particules au moyen d'un affineur industriel (marque Inotec type "I175CDI-75D"). Par « fines particules », on entend des particules dont la taille est comprise entre 50 et 1000 nm, avec deux populations, la première dont les tailles sont comprises entre 50 et 200 nm, la seconde dont les tailles sont comprises entre 600 et 1000 nm.

Le broyat est ensuite pressé au moyen d'une presse à bande industrielle de marque Flottweg type "B FRU 800 HK" à un débit d'environ 1 tonne/heure.

Cette étape permet la séparation de la phase solide (marc) de la phase liquide (jus). Le rendement en jus obtenu est de 75%.

Les 750 kg de jus brut obtenus sont ensuite clarifiés au moyen d'un clarificateur à assiettes de marque Flottweg type "AC 2000".

On obtient ainsi 710 kg d'un jus clair à 3.10 % de masse sèche (95 à 98% de rendement en masse) et une crème (2 à 5% en masse).

Par la suite, le jus clair est ultrafiltré sur une membrane céramique (Tami Industries) de 15 KDa.

On obtient ainsi un perméat et un rétentat. Le perméat est conservé jusqu'à l'obtention de 640 kg de jus de filtration (91% de rendement en volume) à 2.2% de matière sèche.

Le jus de filtration (perméat) est alors séché par lyophilisation après concentration par évaporation.

La concentration est réalisée sur un évaporateur simple effet (EVA 1000, Pignat) avec les paramètres suivants: recirculation forcée, débit d'alimentation 10L/h, pression vapeur de 1 bar, pression de vide de 0,3 bar et température d'évaporation de 90°C.

Une première concentration est réalisée avec un débit d'eau évaporée de 8 L/h et le °brix monte de 5.5 (égal à une concentration de matière sèche de 4.5%) à 14.7.
Cette solution est ensuite concentrée une deuxième fois avec un débit d'eau évaporée de 5-6 L/h et le °brix monte jusqu'à 34. La concentration de matière sèche de la solution est déterminée à 38.4%.

La lyophilisation est ensuite réalisée à l'aide d'un appareil Bioblock scientific (modèle CHRIST alpha 1-4 LSC) à une température de congélation de -80°C qui est également la température minimale au cours de cette étape.

La poudre obtenue est ensuite broyée avec un broyeur planétaire MiniMill de marque Philips. Le produit a été introduit dans des bols de broyage (10 g de produit dans chaque bol de broyage avec 4 billes en zircone). L'ensemble a été mis en rotation pendant 15 minutes à la vitesse 10.

On obtient ainsi 14 kg de poudre d'extrait d'algues.

### Exemple 2 : Détermination de la taille des polysaccharides polyanioniques sulfatés et non sulfatés d'un extrait d'algues selon l'invention par GPC (Gel Permeation Chromatography)

L'extrait d'algues selon l'invention et préparé suivant l'exemple 1 est ultrafiltré sur une membrane de 1000 Da et est dissous à une concentration de 0.5 g/L dans de l'eau. Il est ensuite injecté sur deux colonnes shodex 802 et 803 placées en série (domaine de fractionnement de la colonne 802 : 4.10³ Da et de la colonne 803: 1.7.10⁵ Da). L'éluant utilisé est le nitrate de sodium 0.1 M avec de l'azoture de sodium 0.2% à un débit de 0.5 ml/min. La détection est réalisée via un réfractométre Wyatt et un détecteur à diffusion de la lumière 18 angles Wyatt. Les dn/dc sont pris égaux à 0.150 mL/g.

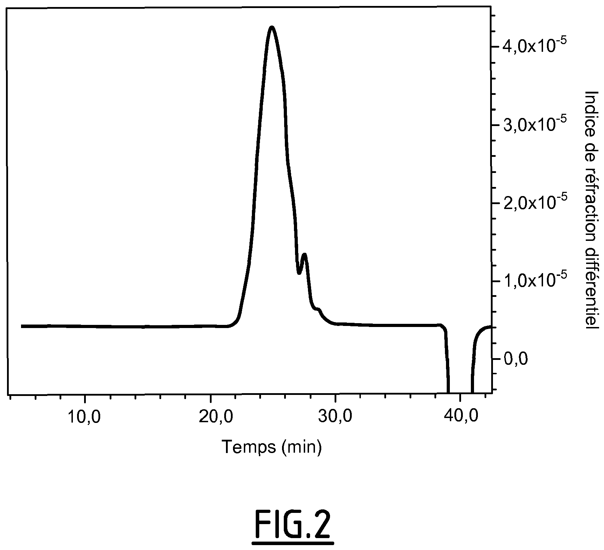

Le chromatogramme détecté par le refractomètre est présenté en figure 2.

On obtient une taille moyenne des polysaccharides d'un extrait d'algues selon l'invention_de 4.4 KDa.

### Exemple 3 : Détermination de la composition d'un extrait d'algues selon l'invention

L'extrait d'algues selon l'invention et préparé selon l'exemple 1 est purifié par ultrafiltration frontale dans des cellules amicon fonctionnant sous agitation. Une membrane en cellulose régénérée de seuil de coupure de 1000 Da est utilisée. 572.1 mg d'échantillon de l'extrait d'algues selon l'invention sont dissous dans 150 ml d'eau ultrapure milli-Q. Cinq litres d'eau sont utilisés pour éliminer toutes les molécules de masse inférieure à 1000 Da. Le rétentat est lyophilisé. 117 mg d'échantillon sont pesés. Le rendement de l'ultrafiltration est donc de 20.5% (p/p). Les analyses suivantes ont été effectuées sur les échantillons ultrafiltrés.

Le ratio des monosaccharides constitutifs des polysaccharides de l'extrait d'algues selon l'invention est déterminé selon la méthode de Kamerling (Kamerling *et al*., 1975) modifiée par Montreuil (Montreuil *et al*., 1986). L'identification et le dosage des monosaccharides nécessitent une hydrolyse par méthanolyse du polymère, de façon à n'obtenir que des monomères. Les résidus glycosidiques sont ensuite triméthylsilylés afin de les rendre volatils. Ils sont ainsi identifiés et dosés par chromatographie en phase gazeuse sous forme de méthylglycosides *O*triméthylsilylés.

Les réactifs suivants sont utilisés :
- Solution de méthanol/HCl 3N (Supelco) ;
- Carbonate d'argent;
- Myo-inositol ;
- Pyridine ;
- Réactif Sylon BFT (BSTFA +TMCS 99 :1) (Supelco) ; et
- Dichlorométhane.

Le mode opératoire est le suivant : 400 µg de l'extrait d'algues selon l'invention préparé tel que mentionné ci-dessus et 50 µg de myo-inositol sont placés dans un bain à sec en présence de 500 µl d'un mélange méthanol/acide chlorhydrique 3 N (Supelco) pendant 4 heures à 100°C. Après refroidissement à température ambiante, le méthanolysat est neutralisé à l'aide de carbonate d'argent. Les échantillons sont centrifugés 15 minutes à 3000 tr/min et le surnageant est évaporé sous jet d'azote. Les composés sont ensuite dissous dans 80 µl de pyridine et incubés durant 25 minutes à 80°C avec 80 µl de sylon (BSTFA : TMCS, 99:1, Supelco). Après évaporation douce des réactifs en excès sous jet d'azote, les méthylglycosides triméthylsilylés sont repris dans 500 µl de dichlorométhane puis injectés en chromatographie en phase gazeuse (injection *in*-colonne, détecteur FID : ionisation de flamme). Le gaz vecteur est l'azote. La colonne, de type HP-5MS (30 m, 0.25 mm de diamètre interne), est apolaire. Le programme de montée en température est le suivant: 120°C maintenus pendant 1 minute, puis un gradient de 1.5°C/min jusqu'à 180°C, suivi d'un gradient de 2°C/min jusqu'à 200°C.

Chaque monosaccharide est identifié par comparaison de ses temps de rétention relatifs par rapport à l'étalon interne, avec ceux des monosaccharides purs traités dans les mêmes conditions. Un coefficient de réponse est calculé pour chaque monosaccharide par rapport à l'étalon interne afin de définir la proportion de chaque monosaccharide au sein des polysaccharides de l'extrait d'algues selon l'invention.

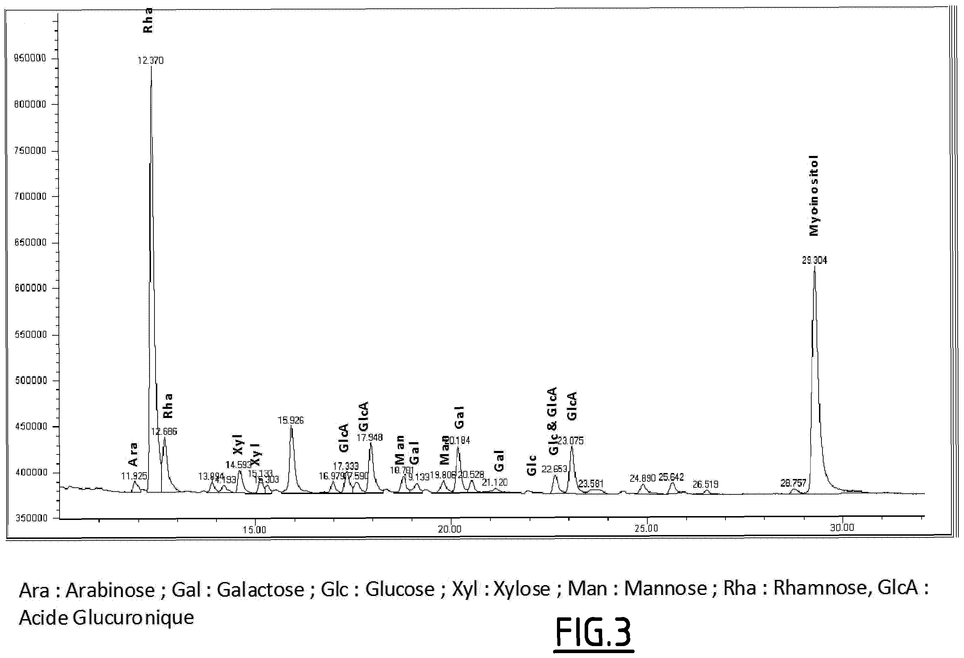

Les résultats obtenus sont présentés dans le tableau 1 ci-dessous et en figure 3.

**Tableau 1 : Composition de l'extrait d'algues selon l'invention obtenue après l'analyse de dérivés triméthylsilylés par chromatographie en phase gazeuse, exprimée en poids par rapport au poids total de l'extrait d'algues; avec Ara: Arabinose ; Gal: Galactose ; Glc: Glucose ; Xyl: Xylose ; Man: Mannose ; Rha: Rhamnose, GlcA: Acide Glucuronique.**

| Echantillon | % en poids de l'ultrafiltrat | Rendement ultrafiltration | % en poids de l'extrait brut |
|---|---|---|---|
| Ara | 0,6 | 20,50% | 0,123 |
| Gal | 1,3 | 20,50% | 0,267 |
| Glc | 0,1 | 20,50% | 0,021 |
| Xyl | 2,45 | 20,50% | 0,502 |
| Man | 0,45 | 20,50% | 0,092 |
| Rha | 39,6 | 20,50% | 8,118 |
| GlcA | 12,9 | 20,50% | 2,645 |

### Exemple 4 : Evaluation de l'activité antibactérienne de l'extrait d'algues selon l'invention

### Matériels et méthodes

Afin de déterminer l'effet antibactérien d'un extrait d'algues EA1 selon l'invention, la concentration minimale inhibitrice de celui-ci est déterminée par une technique en milieu solide en milieu gélosé.

### Souches bactériennes

Les souches bactériennes testées sont indiquées dans le tableau 2 ci-dessous.

**Tableau 2 :**

| **Nom** | **N°** | **Origine** |
|---|---|---|
| *Salmonella Typhimurium* | CIRMBP-940 ATCC 14028 | Bovine, Septicémie |
| *Escherichia coli* | CIRMBP-945 K88 | Porc |
| *Staphylococcus aureus* | CIRMBP-476 | Mammite vache |
| *Listeria monocytogenes* | CIRMBP-711 EGD | Tissus lapin |
| *Escherichia coli* | CIRMBP-096 | Fèces poulet |

Les paramètres de croissance de ces bactéries sont connus sauf pour la souche *E. coli* K88 CIRMBP-945 pour laquelle la courbe de croissance a été établie au préalable.

### Réactifs

Les réactifs suivants sont utilisés :
- Bouillon MH et TSB,
- Eau milliQ ultra pure stérile,
- Eau physiologique tamponnée et tween 80 à 0,5%,
- Boite TSA (Trypticase Soy Agar) ou BHI (Brain Heart Infusion), et
- Flacon de MH gélose.

### Matériel

- Micropipette 1mL avec cônes stériles,
- Micropipette multicanal de 20-300µL avec cônes stériles,
- Pipettes plastiques graduées 1mL stériles,
- Boites de Pétri stériles,
- Etuve 37°C,
- Incubateur agitant 37°C, et
- Tubes Falcon 50mL.

### Extraits d'algues

Un extrait d'algues selon l'invention nommé EA1 et préparé selon l'exemple 1 est tout d'abord pesé puis dissous dans 15 mL d'eau physiologique stérile et agité pendant 15 minutes. La quantité totale de la solution est ensuite ajustée à 20 mL avec l'eau physiologique.

La solution est ensuite séparée en deux et stérilisée suivant deux méthodes différentes. Selon la première méthode, l'extrait d'algues EA1 est autoclavé pendant 15 minutes à 110°C.

Selon la seconde méthode, l'extrait d'algues EA1 dissous est filtré sur un filtre de 0,2 µm. Les extraits d'algues ainsi préparés ont une concentration de 500 g/L.

En parallèle, une solution de gentamicine à 40 mg/L (Gentamicin Sulfate N°17-518Z (Lonza)) est préparée pour servir de témoin positif.

### Mode opératoire

### Préparation avant la manipulation

- Identification des tubes pour faire le mélange milieu/produit à tester,
- Schéma de boite: emplacement des stries/souches à photocopier et découper puis scotcher sur le fond des boites,
- Tubes pour les précultures,
- Tubes et erlen pour la préparation des cultures.

### 1er jour

Le milieu MH est réchauffé pour qu'il soit à la température adéquate avant de le manipuler car il se refroidit très vite et donc se solidifie.

Les milieux gélosés pour les boites témoin (témoins de croissance et de stérilité du milieu) sont préparés en chauffant un flacon de gélose MH jusqu'à liquéfaction et en coulant 4 fois 15 mL de milieu dans des boites de Pétri stériles (3 pour les témoins de croissance et 1 pour le témoin de stérilité).

Des milieux gélosés pour les boites correspondant au témoin de croissance avec diluant (eau Ultra pure stérile) sont préparés en distribuant dans chacun des tubes falcon l'eau Ultra pure stérile. A partir du flacon de gélose MH, le milieu est distribué dans des tubes. Les géloses sont ensuite mélangées doucement et coulées dans une boite de Pétri. Le tube avec le plus d'eau Ultra pure est fait en double pour le contrôle de la stérilité de l'eau Ultra pure.

Le tableau 3 ci-dessous précise le mode opératoire suivi.

**Tableau 3**

| Tubes n° | Quantité d'eau Ultra pure | Quantité du milieu | Facteur de dilution |
|---|---|---|---|
| 1 | 3,75 mL | 11,25 mL | 1/4 |
| 2 | 3,75 mL | 11,25 mL | 1/4 |
| 3 | 1,875 mL | 13,1 mL | 1/8 |
| 4 | 937,5 µL | 14 mL | 1/16 |
| 5 | 468,75 µL | 14,5 mL | 1/32 |
| 6 | 234,4 µL | 14,8 mL | 1/64 |
| 7 | 117,2 µL | 14,9 mL | 1/128 |
| 8 | 58,61 µL | 14,95 mL | 1/256 |

Des milieux gélosés pour les boites correspondant au témoin de croissance avec de la gentamicine sont préparés en distribuant la gentamicine dans chacun des tubes Falcon.

A partir du flacon de gélose MH, le milieu est distribué dans des tubes puis les géloses sont mélangées doucement et coulées dans une boite de Pétri. Le tube avec le plus de gentamicine est fait en double pour contrôler sa stérilité.

Le tableau 4 ci-dessous précise le mode opératoire suivi.

**Tableau 4**

| Tubes n° | Quantité d'eau Ultra pure | Quantité du milieu | Concentration en Gentamicine |
|---|---|---|---|
| 1 | 3,75 mL | 11,25 mL | 10 mg/L |
| 2 | 3,75 mL | 11,25 mL | 10 mg/L |
| 3 | 1,875 mL | 13,1 mL | 5 mg/L |
| 4 | 937,5 µL | 14 mL | 2,5 mg/L |
| 5 | 468,75 µL | 14,5 mL | 1,25 mg/L |
| 6 | 234,4 µL | 14,8 mL | 0,63 mg/L |
| 7 | 117,2 µL | 14,9 mL | 0,31 mg/L |
| 8 | 58,61 µL | 14,95 mL | 0,16 mg/L |
| 89 | 29.3 µL | 14,95 mL | 0,08 mg/L |

Des milieux gélosés pour les boites correspondant à l'extrait d'algues à tester sont préparés. Deux séries seront faites : une avec l'extrait d'algues filtré, l'autre avec l'extrait d'algues autoclavé. L'extrait d'algues filtré ou autoclavé est distribué dans chacun des tubes Falcon. A partir du flacon de gélose MH, le milieu est distribué dans des tubes. Les géloses sont ensuite mélangées doucement et coulées dans une boite de Pétri. Le tube avec le plus d'extrait d'algues sera fait en double pour contrôler sa stérilité.

Le tableau 5 ci-dessous précise le mode opératoire suivi.

**Tableau 5**

| Tubes n° | Quantité d'eau Ultra pure | Quantité du milieu | Concentration en extrait d'algues |
|---|---|---|---|
| 1 | 3,75 mL | 11,25 mL | 125,3 mg/L |
| 2 | 3,75 mL | 11,25 mL | 125,3 mg/L |
| 3 | 1,875 mL | 13,1 mL | 62,2 mg/L |
| 4 | 937,5 µL | 14 mL | 31,1 mg/L |
| 5 | 468,75 µL | 14,5 mL | 15,5 mg/L |
| 6 | 234,4 µL | 14,8 mL | 7,8 mg/L |
| 7 | 117,2 µL | 14,9 mL | 3,9 mg/L |
| 8 | 58,61 µL | 14,95 mL | 1,9 mg/L |

L'ensemencement des précultures est réalisé à partir d'un congelât de la souche à tester à 25% en glycérol, gratté et aspiré avec une pipette de 1mL et mis dans 9mL de milieu de culture adéquat. En parallèle, la pureté de la souche est vérifiée en faisant un isolement sur boite de gélose. L'incubation se déroule la nuit à 37°C avec ou sans agitation selon la souche.

Les conditions de cultures selon la souche utilisée sont indiquées dans le tableau 6 ci-dessous.

**Tableau 6**

| **Souches** | **Milieu** | **Agitation** |
|---|---|---|
| *Escherichia coli K88* | MH | sans |
| *Escherichia coli 096* | MH | sans |
| *Staphylococcus aureus* | MH | sans |
| *Salm. Typhimurium* | TSB | avec |
| *Listeria monocytogenes* | TSB | avec |

### 2ème jour

L'ensemencement des milieux de culture incubés est déterminé et des dilutions des cultures sont effectuées pour arriver à des concentrations finales de 10⁵ UFC/mL.

Les boites sont ensuite ensemencées. Sur les boites préalablement identifiées, l'ensemencement est réalisé en strie à l'aide d'une anse calibrée de 10µL. La même anse par produit à tester est utilisée, en allant du moins concentré au plus concentré. Une strie dépose donc environ 10³ UFC. L'Incubation est faite en étuve à 37°C.

### Résultats

Le 3^{ème} jour, la lecture des boites est effectuée.

Cette lecture permet de valider les témoins de croissance. Les croissances en eau Ultra pure n'interfèrent pas avec la croissance bactérienne.

Les résultats relatifs aux extraits d'algues selon l'invention sont indiqués dans le tableau 7 ci-dessous.

**Tableau 7 :**

| **Concentration d'extrait d'algues (g/L)** | ***Salmonella Typhimurium* CIRMBP-940 ATCC 14028** | ***Escherichia coli* K88 CIRMBP-945 (porc)** | ***Staphylococcus aureus* CIRMBP-476** | ***Listeria monocytogenes* EGD CIRMBP-711** | ***Escherichia coli* CIRMBP-096 (poulet)** |
|---|---|---|---|---|---|
| 125,3 | - | - | - | - | - |
| 62,2 | - | - | - | - | + |
| 31,1 | + | + | - | "+ faible" | + |
| 15,5 | + | + | - | + | ++ |
| 7,8 | + | + | - | + | + |
| 3,9 | + | + | - | + | + |
| 1,9 | + | + | + | + | + |
| CMI | 31,1 <CMI≤62,2 | 31,1<CMI≤62,2 | 1,9<CMI≤3,9 | 31,1<CMI≤62,2 | 62,2<CMI≤125,3 |

Cette expérience est répétée une seconde fois en utilisant le mode opératoire précédemment décrit et les résultats obtenus sont indiqués dans le tableau 8 ci-dessous.

**Tableau 8**

| **Concentration d'extrait d'algues (g/L)** | ***Salmonella Typhimurium* CIRMBP-940 ATCC 14028** | ***Escherichia coli* K88 CIRMBP-945 (porc)** | ***Staphylococcus aureus* CIRMBP-476** | ***Listeria monocytogenes* EGD CIRMBP-711** | ***Escherichia coli* CIRMBP-096 (poulet)** |
|---|---|---|---|---|---|
| 125,3 | - | - | - | - | - |
| 62,2 | + | "+ faible" | - | - | - |
| 31,1 | + | + | - | + | "+ faible" |
| 15,5 | + | + | - | + | + |
| 7,8 | + | + | - | + | + |
| 3,9 | + | + | - | + | + |
| 1,9 | + | + | + | + | + |
| CMI | 62,2<CMI≤9125,3 | 62,2<CMI≤125,3 | 1,9<CMI≤3,9 | 31,3<CMI≤62,6 | 31,1<CMI≤62,2 |

Dans les tableaux 7 et 8 ci-dessus, le signe « -» dénote l'absence de colonies et le signe « + » leur présence. CMI signifie « concentration minimale inhibitrice ».

Le tableau 9 représente un récapitulatif des différents résultats obtenus relatifs à la concentration minimale inhibitrice d'extrait d'algues selon l'invention observée lors de ces deux expériences pour chacune des souches testées.

**Tableau 9**

| | **Gélose essai 1** | **Gélose essai 2** | **Conclusion (estimation CMI)** |
|---|---|---|---|
| *Escherichia coli poulet* | 62,2<CMI≤ 125,3 | 31,3<CMI≤62,5 | CMI = 62,35 |
| *Escherichia coli porc* | 31,1<CMI≤62,2 | 62,5<CMI≤125 | CMI = 63 |
| *Salmonella Typhimurium* | 31,1<CMI≤62,2 | 62,5<CMI≤125 | CMI = 63 |
| *Listeria monocytogenes* | 31,1<CMI≤62,2 | 31,3<CMI≤62,6 | 31,3<CMI≤62,6 |
| *Staphylococcus aureus* | 1,9<CMI≤3,9 | 1,9<CMI≤3,9 | CMI = 4 |

### Conclusion

L'administration d'un extrait d'algues selon la présente invention met ainsi en évidence des effets antibactériens du produit, notamment vis-à-vis des bactéries *Staphylococcus aureus, Listeria monocytogenes, Escherichia coli* et *Sallmonella typhimurium.*

### Exemple 5 : Evaluation complémentaire de l'activité antibactérienne de l'extrait d'algues selon l'invention

### Objectifs :

Déterminer les CMI (Concentration minimal inhibitrice) d'un extrait d'algues selon l'invention EA2 sur un panel de quarante souches à Gram négatif et Gram positif.

### Principe :

La détermination de la CMI est réalisée par la méthode de dilution en milieu gélosé. Cette méthode consiste à incorporer l'extrait d'algues selon l'invention dans le milieu gélosé en boîtes de Pétri, en réalisant une gamme de concentrations en progression géométrique de raison deux.

Les souches bactériennes pures sont ensemencées à la surface de chaque boîte en utilisant un ensemenceur multipoints (Denley) qui permet l'ensemencement simultané de 21 souches sous forme de spots déposés à la surface de chaque boîte de la série. L'inoculum doit être d'environ 104 bactéries/ spot (Référence : Comité de l'Antibiogramme de la Société Française de Microbiologie (CASFM)).

### Matériel et méthodes

### Extrait d'algues

Un extrait d'algues selon l'invention nommé EA2 et préparé selon l'exemple 1 est utilisé. Il est autoclavé 15 minutes à 110°C dans 5 flacons de 15 mL.

### Souches bactériennes

### Souches non exigeantes

Les souches non exigeantes testées sont indiquées dans le tableau 10 ci-dessous :

**Tableau 10**

| N° | Nom |
|---|---|
| CIRMBP-949 | E. coli hémolytique |
| CIRMBP-0552 | Staphylococcus chromogenes |
| LH 2014 / 1682 | E COLI 078K80 |
| LH 2014 /903 | E COLI 02 |
| LH 2014 / 1423 | E COLI 01 |
| SA 2010/8843 | S Enteritidis |
| SA 2014/7652 | S Typhimurium |
| MA 61985 | E coli CS31A |
| MA61391 | E coli K99 |
| MA58166 | E coli K85 |
| MA 61283 | E coli F17 |
| MA 61065,2 | E coli K88 (F4) |
| MA62166-2 | Staphylococcus aureus bovins |
| MA61990 | Staphylococcus aureus poulet |
| MA61899-3 | Serratia marcescens |
| SA 2014/8625 | S Hadar |
| SA 2012/33827 | S Infantis |
| Souche réf Resalab | S Virchow |
| LH 2013-5643 | Staphylococcus aureus volaille |
| LH2014-2855 | Klebsiella pneumoniae |

### Réactifs

- Bouillon Brain Heart Infusion (BHI) en tube contenant 5 ml,
- Boite BHI et gélose au sang,
- Gélose de Mueller-Hinton en tube contenant 18 ml,
- Eau physiologique stérile en tube API NaCl 0.85% (ref 20070 Biomerieux)
- Eau physiologique stérile en tube contenant 4,5 ml,
- Eau physiologique stérile en flacon,
- Eau UP stérile en flacon,
- EA2.

### Matériels

- Anse,
- Pipettes graduées stériles,
- Tubes stériles,
- Boîtes de Pétri stériles (diamètre 90 mm),
- Etuve à 37°C,
- Agitateur de type Vortex,
- Densitomètre Densimat BioMérieux,
- Bain-Marie à 50°C,
- Inoculateur multipoint Denley et accessoires (plaque téflon stérile + jeu de tiges stériles).

### Protocole

Le premier jour, chaque souche est ensemencée à partir du congelât à -80°C, dans 5mL de bouillon BHI puis Incubée 18h à 37°C.

Le second jour, les bouillons sur BHI ou gélose au sang pour les Staphylocoques sont isolés puis incubés 18h à 37°C.

Pour le 3^{ème} jour, les tubes MH à température ambiante sont sortis, les 3mL d'eau UP sont distribués, les boites de Pétri identifiées, le schéma de dépôt fait et la solution EA2 maintenue à 4°C.

Le 3^{ème} jour, la pureté des souches est vérifiée, et la gamme de dilution de l'extrait d'algues EA2 préparée dans des tubes stériles comme indiqué ci-dessous dans le tableau 11.

**Tableau 11**

| Solution mère d'EA2 à 1000g/L | Eau UP stérile | Titre obtenu en g/L | Concentration finale dans la gélose de Mueller-Hintonen g/L |
|---|---|---|---|
| 3 ml | 3 ml | 500 | 50 |

| Puis dilution en cascade : 3ml de la solution fille précédente dans 3ml d'eau milliQ | | | |
|---|---|---|---|
| | 3ml | 250 | 25 |
| | 3ml | 125 | 12.5 |
| | 3ml | 62.5 | 6.3 |
| | 3ml | 31.25 | 3.1 |
| | 3ml | 15.6 | 1.6 |
| | 3ml | 7.8 | 0.8 |

### Préparation des boites de gélose Mueller-Hinton + EA2

Les tubes de MH sont chauffés dans un bécher à l'eau bouillante. Dès que la gélose est complètement fondue, les tubes sont mis au bain-marie à 50°C. 2 ml de chaque dilution de la gamme d'EA2 est mise dans une boîte de Pétri. 18 ml de gélose de Mueller-Hinton sont ajoutés, bien mélangés et laissés solidifier. Les boîtes sont séchées 30 minutes sous hotte à flux laminaire. Une boîte de gélose de Mueller-Hinton sans EA2 est préparée qui servira de boîte témoin avec 2 ml d'eau UP stérile.

### Préparation de l'inoculum

La colonie est prélevée pour préparer une suspension à 0.5McF dans les tubes API ajusté à l'aide du densimat.

N.B : La valeur de 0,5 Mac Farland correspond à une concentration bactérienne d'environ 108 bactéries/ml.

Une dilution au 1/10ème de cette suspension (0,5 Mac Farland) est réalisée en mettant 0,5 ml dans un tube contenant 4,5 ml d'eau physiologique stérile (cette suspension correspond à environ 107 bactéries/ml). 0.5mL de l'inoculum est déposé à la suite dans chaque godet de la plaque en téflon.

### Ensemencement des boites

Un inoculateur multipoint « Denley » est utilisé, qui permet l'ensemencement simultané de 21 souches bactériennes en spots sur chaque boîte. Chaque tige dépose de 2 à 5 µl/spot (soit environ 104 bactéries/spot).

La boite de Pétri est placée à gauche, la plaque de téflon à droite après l'avoir tapotée légèrement pour remettre en suspension les bactéries. Une boîte de Gélose de Mueller-Hinton sans EA2 est ensemencée qui servira de boîte témoin. L'inoculation est faite de la concentration la plus faible à la plus forte.

Changer le portoir de tiges entre chaque série. Les boîtes sont laissées à la température ambiante pendant environ 30 minutes puis incubées 18 à 24 heures à l'étuve à 37°C.

Le 4^{ème} jour, la croissance bactérienne est observée au niveau de chaque spot.

La CMI est la plus faible concentration d'EA2 pour laquelle il n'y a aucune croissance visible de la souche bactérienne testée.

### Souches exigeantes

Les souches testées sont présentées dans le tableau 12 ci-dessous.

**Tableau 12**

| N° | Nom |
|---|---|
| CIRMBP-0948 | Streptococcus dysgalactiae ssp dysgalactiae |
| CIRMBP-0619 | Streptococcus dysgalactiae |
| CIRMBP-0635 | Streptococcus dysgalactiae |
| CIRMBP-0637 | S. uberis |
| LH 2013/1796 | Listeria monocytogenes |
| CIRMBP-976 | Listeria monocytogenes |
| MA56030 | Streptocoque dysgalactiae |
| MA61918 | Streptocoque uberis n°2 |
| MA60160 | Streptocoque suis II |
| CIRMBP-947 | Mannheimia |
| CIRMBP-0437 | Pasteurella multocida subsp multocida SOUCHE TYPE |
| CIRMBP-0438 | Pasteurella multocida subsp gallicida SOUCHE TYPE |
| CIRMBP-873 (LVT 51) | Pasteurella multocida subsp septica |
| MA61322 | Pasteurella multocida |
| LH2012/108 | Mannheimia haemolytica |
| LH 2011/5575 | Actinomyces pyogenes |
| MA60111 | Bordetella bronchiseptica |
| CIRMBP-975 | Corynebacterium bovis |
| LH 2014 / 429 | Erysipelothrix |
| LH2014/1921 | Enterococcus cecorum |

| | |
|---|---|
| + souche témoin interne: CIRMBP-711 : *L. monocytogenes* 31,1<CMI≤62,2 g/L. | |

### Réactifs

- Bouillon Brain Heart Infusion (BHI) en tube contenant 5 ml,
- Sérum de cheval,
- Boite TSA-YE sérum et gélose au sang,
- Gélose de Mueller-Hinton en tube contenant 18 ml,
- Eau physiologique stérile en tube API NaCl 0.85% (ref 20070 Biomerieux),
- Eau physiologique stérile en tube contenant 4,5 ml,
- Eau physiologique stérile en flacon,
- Eau UP stérile en flacon,
- EA2.

### Matériels

- Anse,
- Pipettes graduées stériles,
- P1000 + cônes à filtres stériles,
- Tubes stériles,
- Boîtes de Pétri stériles (diamètre 90 mm),
- Etuve à 37°C+ 5% CO₂,
- Agitateur de type Vortex,
- Densitomètre Densimat BioMérieux,
- Bain-Marie à 50°C,
- Inoculateur multipoint Denley + Accessoires (plaque téflon stérile + jeu de tiges stériles).

### Protocole

Chaque souche est ensemencée à partir du congelât à -80°C, dans le milieu approprié, comme indiquée dans le tableau 13 ci-dessous.

**Tableau 13**

| **N° CIRM** | **Nom** | **Milieu bouillon J1** | **Milieu gélosé J2** |
|---|---|---|---|
| CIRMBP-0948 | Streptococcus dysgalactiae ssp dysgalactiae | BHI | GS |
| CIRMBP-0619 | Streptococcus dysgalactiae | BHI | GS |
| CIRMBP-0635 | Streptococcus dysgalactiae | BHI | GS |
| CIRMBP-0637 | S. uberis | BHI | GS |
| LH 2013/1796 | Listeria monocytogenes | BHI | GS |
| CIRMBP-976 | Listeria monocytogenes | BHI | GS |
| MA56030 | Streptocoque dysgalactiae | BHI | GS |
| MA61918 | Streptocoque uberis n°2 | BHI | GS |
| MA60160 | Streptocoque suis II | BHI | GS |
| CIRMBP-947 | Mannheimia | BHI + 5% serum | TSA-YE serum |
| CIRMBP-0437 | Pasteurella multocida subsp multocida SOUCHE TYPE | BHI + 5% serum | GS |
| CIRMBP-0438 | Pasteurella multocida subsp gallicida SOUCHE TYPE | BHI + 5% serum | GS |
| CIRMBP-873 (LVT 51) | Pasteurella multocida subsp septica | BHI + 5% serum | GS |
| MA61322 | Pasteurella multocida | BHI + 5% serum | GS |
| LH2012/108 | Mannheimia haemolytica | BHI + 5% serum | TSA-YE serum |
| LH 2011/5575 | Actinomyces pyogenes | BHI + 5% serum | GS |
| MA60111 | Bordetella bronchiseptica | BHI + 5% serum | GS |
| CIRMBP-975 | Corynebacterium bovis | BHI + 5% serum | GS |
| LH 2014 / 429 | Erysipelothrix | BHI + 5% serum | GS |
| LH2014/1921 | Enterococcus cecorum | BHI | GS |
| CIRMBP-711 | Listeria monocytogenes | BHI | GS |

Elles sont ensuite incubées 18h à 37°C + 5% CO₂.

L'extrait d'algues EA2 est préparé tel qu'indiqué dans le tableau 14 ci-dessous.

**Tableau 14**

| Solution mère d'UA2 à 1000g/L | Eau UP stérile | Titre obtenu en g/L | Concentration finale dans la gélose de Mueller-Hintonen |
|---|---|---|---|
| 3 ml | 3 ml | 500 | 50 g/L |

| Puis dilution en cascade : 3ml de la solution fille précédente dans 3ml d'eau milliQ | | | |
|---|---|---|---|
| | 3ml | 250 | 25 g/L |
| | 3ml | 125 | 12.5 g/L |
| | 3ml | 62.5 | 6.3 g/L |
| | 3ml | 31.25 | 3.1 g/L |
| | 3ml | 15.6 | 1.6 g/L |
| | 3ml | 7.8125 | 781.25 mg/L |
| | 3ml | 3.90625 | 390.6 mg/L |
| | 3ml | 1.953125 | 195.3 mg/L |
| Prendre 300µL de la solution à 15.6 g/L | 2.7ml | 1.56 | 156 mg/L |
| Prendre 300µL de la | 2.7ml | 0.78 | 78 mg/L |
| solution à 7.8 g/L | | | |
| Prendre 300µL de la solution à 3.9 g/L | 2.7ml | 0.39 | 39 mg/L |

### Préparation des boites de gélose Mueller-Hinton + UA2

Les tubes de MH sont chauffés dans un bécher à l'eau bouillante. Dès que la gélose est complètement fondue, les tubes sont mis au bain-marie à 50°C. 1 mL de sérum de cheval est mis dans chaque boîte de Pétri. 2.1 ml de chaque dilution de la gamme d'EA2 sont mis dans une boîte de Pétri. 18 ml de gélose de Mueller-Hinton sont ajoutés, bien mélangés et laissés solidifier. Les boîtes sont séchées 30 minutes sous hotte à flux laminaire.

Une boîte de gélose de Mueller-Hinton sans extrait d'algues EA qui servira de boîte témoin est préparée avec 3 ml d'eau UP stérile.

### Préparation de l'inoculum

La colonie est prélevée pour préparer une suspension à 0.5McF dans les tubes API ajusté à l'aide du densimat.

N.B : La valeur de 0,5 Mac Farland correspond à une concentration bactérienne d'environ 108 bactéries/ml.

Une dilution au 1/10ème de cette suspension (0,5 Mac Farland) est réalisée en mettant 0,5 ml dans un tube contenant 4,5 ml d'eau physiologique stérile (cette suspension correspond à environ 107 bactéries/ml).

0.5mL de *l'inoculum* est déposé à la suite dans chaque godet de la plaque en téflon.

### Ensemencement des boites

Un inoculateur multipoint « Denley » qui permet l'ensemencement simultané de 21 souches bactériennes en spots sur chaque boîte est utilisé. Chaque tige dépose de 2 à 5 µl/spot (soit environ 104 bactéries/spot). La boite de Pétri est placée à gauche, la plaque de téflon à droite après l'avoir tapotée légèrement pour remettre en suspension les bactéries. Une boîte de gélose de Mueller-Hinton sans extrait d'algues EA2 qui servira de boîte témoin est ensemencée. L'inoculation est faite de la concentration la plus faible à la plus forte.

Les boîtes sont laissées à la température ambiante pendant environ 30 minutes puis incubées 18 à 24 heures à l'étuve à 37°C. La croissance bactérienne est observée au niveau de chaque spot.

La CMI est la plus faible concentration d'EA2 pour laquelle il n'y a aucune croissance visible de la souche bactérienne testée.

### Résultats

Les résultats sont présentés dans le tableau 14 ci-dessous.

**Tableau 14**

| GRAM | souches | N° | CMI |
|---|---|---|---|
| - | *Past. m.subsp septica* | CIRMBP-873 | 156 mg/L |
| - | *Pasteurella multocida subsp gallicida* ST | CIRMBP-0438 | 390.6 mg/L |
| - | *Mannheimia haemolytica* | LH2012/108 | 390.6 mg/L |
| - | *Pasteurella multocida* | MA61322 | 1.56 g/L |
| - | *Pasteurella multocida subsp multocida* ST | CIRMBP-0437 | 3.125 g/L |
| - | *Mannheimia* | CIRMBP-947 | 3.125 g/L |
| + | *Erysipelothrix* | LH 2014 / 429 | 3.125 g/L |
| + | *Staphylococcus aureus* volaille | LH 2013-5643 | 3.125 g/L |
| + | *Staphylococcus aureus* bovins | MA62166-2 | 3.125 g/L |
| + | *Staphylococcus aureus* poulet | MA61990 | 3.125 g/L |
| + | Témoin : *S. aureus* | CIRMBP-476 | 3.125 g/L |
| + | *Streptocoque suis II* | MA60160 | 6.25 g/L |
| + | *Enterococcus cecorum* | LH2014/1921 | 25 g/L |
| + | *Streptococcus dysgalactiae ssp dysgalactiae* | CIRMBP-0948 | 50 g/L |
| + | *Streptococcus dysgalactiae* | CIRMBP-0619 | 50 g/L |
| + | *Streptococcus dysgalactiae* | CIRMBP-0635 | 50 g/L |
| + | *Streptocoque dysgalactiae* | MA56030 | 50 g/L |
| - | *Bordetella bronchiseptica* | MA60111 | 50 g/L |
| + | *Corynebacterium bovis* | CIRMBP-975 | 50 g/L |
| + | *A ctinomyces pyogenes* | LH 2011/5575 | 50 g/L |
| + | *Staphylococcus chromogenes* | CIRMBP-0552 | 50 g/L |
| + | *Streptocoque uberis n°2* | MA61918 | environ 50 g/L |
| + | Témoin : *L. monocytogenes* | CIRMBP-711 | Légèrement > 50 g/L |
| + | *Listeria monocytogenes* | LH 2013/1796 | Légèrement > 50 g/L |
| + | *Listeria monocytogenes* | CIRMBP-976 | Légèrement > 50 g/L |
| - | Serratia marcescens | MA61899-3 | Légèrement > 50 g/L |
| + | *Streptococcus uberis* | CIRMBP-0637 | > 50g/L |
| - | *E. coli* hémolytique | CIRMBP-949 | > 50g/L |
| - | *E. coli* 078K80 | LH 2014 / 1682 | > 50g/L |
| - | *E. coli* 02 | LH 2014 /903 | > 50g/L |
| - | *E. coli* 01 | LH 2014 / 1423 | > 50g/L |
| - | *E coli* CS31A | MA 61985 | > 50g/L |
| - | *E coli* K99 | MA61391 | > 50g/L |
| - | *E coli* K85 | MA58166 | > 50g/L |
| - | *E coli* F17 | MA 61283 | > 50g/L |
| - | *E coli* K88 (F4) | MA 61065,2 | > 50g/L |
| - | *Salm.* Hadar | SA 2014/8625 | > 50g/L |
| - | *Salm.* Infantis | SA 2012/33827 | > 50g/L |
| - | *Salm.* Virchow | Souche | > 50g/L |
| | | référence | |
| - | *Salm.* Enteritidis | SA 2010/8843 | > 50g/L |
| - | *Salm.* Typhimurium | SA 2014/7652 | > 50g/L |
| - | *Klebsiella pneumoniae* | LH2014-2855 | > 50g/L |

### Conclusion

L'administration d'un extrait d'algues selon la présente invention met ainsi en évidence des effets antibactériens du produit vis-à-vis de nombreuses bactéries, à la fois sur des bactéries à Gram positif et des bactéries à Gram négatif, en particulier *Past. m. subsp septica, Pasteurella multocida subsp gallicida ST*, *Mannheimia haemolytica, Pasteurella multocida, Pasteurella multocida subsp multocida ST*, *Mannheimia, Erysipelothrix, Staphylococcus aureus volaille, Staphylococcus aureus bovins, Staphylococcus aureus poulet, Streptocoque suis II.*

## Revendications

1. Extrait d'algues de l'ordre des ulvales, en particulier extrait d'algues vertes de type *Ulva*, comprenant des polysaccharides polyanioniques sulfatés et non sulfatés dont la taille est inférieure à 50 kDa, à l'exclusion de polysaccharides polyanioniques sulfatés et non sulfatés dont la taille est supérieure à 50 kDa, pour son utilisation dans la prévention et/ou le traitement d'une infection bactérienne.

2. Extrait d'algues pour son utilisation selon la revendication 1, dans laquelle lesdits polysaccharides comprennent du mannose et/ou de l'arabinose, en particulier du mannose.

3. Extrait d'algues pour son utilisation selon la revendication 2, dans laquelle lesdits polysaccharides comprennent au moins au 0.005% de mannose et/ou au moins 0.005% d'arabinose, en poids par rapport au poids de la matière sèche totale de l'extrait d'algues.

4. Extrait d'algues pour son utilisation selon la revendication 3, dans laquelle lesdits polysaccharides comprennent du mannose en une quantité allant de 0.01 à 0.50% et/ou de l'arabinose en une quantité allant de 0.01 à 0.5%, en poids par rapport au poids de la matière sèche totale de l'extrait d'algues.

5. Extrait d'algues pour son utilisation selon l'une quelconque des revendications 1 à 4, dans laquelle lesdits polysaccharides comprennent en outre:
- du galactose;
- du glucose;
- du rhamnose;
- du xylose; et
- de l'acide glucuronique.

6. Extrait d'algues pour son utilisation selon la revendication 5, dans laquelle lesdits polysaccharides comprennent :
- de 0.05 à 0.5% de galactose;
- de 0.005 à 0. 5% de glucose;
- de 2 à 15 % de rhamnose;
- de 0.1 à 1% de xylose; et
- de 1 à 7% d'acide glucuronique;
en poids par rapport au poids de la matière sèche totale de l'extrait d'algues.

7. Extrait d'algues pour son utilisation selon l'une quelconque des revendications précédentes, dans laquelle l'infection bactérienne est une infection causée par une bactérie à Gram positif ou à Gram négatif.

8. Extrait d'algues pour son utilisation selon la revendication 7, dans laquelle l'infection bactérienne est une infection causée par une bactérie à Gram positif choisie parmi *Staphylococcus aureus* et *Listeria monocytogenes* ou à Gram négatif choisie parmi *Escherichia coli* et *Sallmonella typhimurium.*

9. Extrait d'algues pour son utilisation selon la revendication 8, dans laquelle l'infection bactérienne est une infection causée par *Staphyloccocus aureus.*

10. Extrait d'algues pour son utilisation selon la revendication 9, dans laquelle l'infection bactérienne est cutanée.

11. Extrait d'algues pour son utilisation selon la revendication 7, dans laquelle l'infection bactérienne est une infection causée par une bactérie choisie parmi *Pasteurella m. subsp septica, Pasteurella multocida subsp gallicida ST, Mannheimia haemolytica, Pasteurella multocida, Pasteurella multocida subsp multocida ST, Mannheimia, Erysipelothrix, Staphylococcus aureus volaille, Staphylococcus aureus bovins, Staphylococcus aureus poulet* et *Streptococcus suis II.*

12. Extrait d'algues pour son utilisation selon l'une quelconque des revendications précédentes, dans laquelle l'extrait d'algues est compris dans une composition pharmaceutique.

13. Extrait d'algues pour son utilisation selon l'une quelconque des revendications 1 à 12, dans laquelle les polysaccharides polyanioniques sulfatés et non sulfatés ont une taille inférieure ou égale à 15 kDa.

14. Extrait d'algues pour son utilisation selon l'une quelconque des revendications 1 à 6, dans laquelle ledit extrait d'algues est susceptible d'être obtenu par un procédé de préparation comprenant les étapes suivantes:
a) les algues sont lavées et dessablées ;
b) lesdites algues sont broyées ;
c) la phase solide du broyat est séparée de sa phase liquide ;
d) ladite phase liquide est clarifiée ;
e) le jus obtenu à l'étape d) est ultrafiltré sur une membrane de moins de 50 kDa; et
f) le jus de filtration obtenu à l'étape e) est concentré puis séché.

15. Extrait d'algues pour son utilisation selon la revendication 14, dans laquelle le jus obtenu à l'étape d) du procédé de préparation est ultrafiltré sur une membrane de 15 kDa ou moins.

16. Extrait d'algues pour son utilisation selon l'une des revendications 14 ou 15, dans laquelle l'extrait d'algues obtenu à l'étape f) du procédé de préparation est purifié, notamment par ultrafiltration.

## Patentansprüche

1. Algenextrakt der Ordnung von Ulvales, insbesondere Grünalgenextrakt vom *Ulva*-Typ, umfassend sulfatierte und nicht-sulfatierte polyanionische Polysaccharide, deren Größe weniger als 50 kDa beträgt, mit der Ausnahme von sulfatierten und nicht-sulfatierten polyanionischen Polysacchariden, deren Größe mehr als 50 kDa beträgt, zur Verwendung bei der Vorbeugung und / oder Behandlung einer bakteriellen Infektion.

2. Algenextrakt zur Verwendung nach Anspruch 1, wobei die Polysaccharide Mannose und / oder Arabinose, insbesondere Mannose, aufweisen.

3. Algenextrakt zur Verwendung nach Anspruch 2, wobei die Polysaccharide mindestens 0,005 Gew.-% Mannose und / oder mindestens 0,005 Gew.-% Arabinose aufweisen, bezogen auf das Gewicht der gesamten Trockenmasse des Algenextrakts.

4. Algenextrakt zur Verwendung nach Anspruch 3, wobei die Polysaccharide Mannose in einer Menge im Bereich von 0,01 bis 0,50 Gew.-% und / oder Arabinose in einer Menge im Bereich von 0,01 bis 0,5 Gew.-% aufweisen, bezogen auf das Gewicht der gesamten Trockenmasse des Algenextrakts.

5. Algenextrakt zur Verwendung nach einem der Ansprüche 1 bis 4, wobei die Polysaccharide ferner aufweisen:
- Galactose;
- Glukose;
- Rhamnose;
- Xylose; und
- Glucuronsäure.

6. Algenextrakt zur Verwendung nach Anspruch 5, wobei die Polysaccharide aufweisen:
- 0,05 bis 0,5 Gew.-% Galactose;
- 0,005 bis 0,5 Gew.-% Glukose;
- 2 bis 15 Gew.-% Rhamnose;
- 0,1 bis 1 Gew.-% Xylose; und
- 1 bis 7 Gew.-% Glucuronsäure;
bezogen auf das Gewicht der gesamten Trockenmasse des Algenextrakts.

7. Algenextrakt zur Verwendung gemäß irgendeinem der vorhergehenden Ansprüche, wobei die bakterielle Infektion eine Infektion ist, die durch ein grampositives Bakterium oder ein gramnegatives Bakterium verursacht ist.

8. Algenextrakt zur Verwendung nach Anspruch 7, wobei die bakterielle Infektion eine Infektion ist, die durch ein grampositives Bakterium verursacht ist, ausgewählt aus *Staphylococcus aureus* und *Listeria monocytogenes,* oder die durch ein gramnegatives Bakterium verursacht ist, ausgewählt aus *Escherichia coli* und *Sallmonella typhimurium.*

9. Algenextrakt zur Verwendung nach Anspruch 8, wobei die bakterielle Infektion eine durch *Staphyloccocus aureus* verursachte Infektion ist.

10. Algenextrakt zur Verwendung nach Anspruch 9, wobei die bakterielle Infektion kutan ist.

11. Algenextrakt zur Verwendung nach Anspruch 7, wobei die bakterielle Infektion eine Infektion ist, die durch ein Bakterium verursacht ist, ausgewählt aus *Pasteurella m. subsp septica, Pasteurella multocida subsp gallicida ST, Mannheimia haemolytica, Pasteurella multocida, Pasteurella multocida subsp multocida ST, Mannheimia, Erysipelothrix, Staphylococcus aureus* von Geflügel, bovinem *Staphylococcus aureus, Staphylococcus aureus* von Hähnchen, *Streptococcus suis II.*

12. Algenextrakt zur Verwendung nach irgendeinem der vorhergehenden Ansprüche, wobei der Algenextrakt in einer pharmazeutischen Zusammensetzung enthalten ist.

13. Algenextrakt zur Verwendung nach irgendeinem der Ansprüche 1 bis 12, wobei die sulfatierten und nicht-sulfatierten polyanionischen Polysaccharide eine Größe kleiner als oder gleich 15 kDa haben.

14. Algenextrakt zur Verwendung nach irgendeinem der Ansprüche 1 bis 6, wobei der Algenextrakt durch ein Herstellungsverfahren erhältlich ist, welches die folgenden Schritte aufweist:
a) Waschen und Entsanden der Algen;
b) Zerkleinern der Algen;
c) Trennen der festen Phase des zerkleinerten Materials von seiner flüssigen Phase;
d) Klären der flüssigen Phase;
e) Ultrafiltrieren des in Schritt d) erhaltenen Safts durch eine Membran von weniger als 50 kDa; und
f) Konzentrieren des in Schritt e) erhaltenen Filtrationssafts, anschließend Trocknen.

15. Algenextrakt zur Verwendung nach Anspruch 14, wobei der in Schritt d) des Herstellungsverfahrens erhaltene Saft durch eine Membran von 15 kDa oder weniger ultrafiltriert wird.

16. Algenextrakt zur Verwendung nach einem der Ansprüche 14 oder 15, wobei der in Schritt f) des Herstellungsverfahrens erhaltene Algenextrakt gereinigt wird, insbesondere durch Ultrafiltration.

## Claims

1. Extract of algae of the order Ulvales, in particular extract of green algae of the *Ulva* type, comprising sulfated and non-sulfated polyanionic polysaccharides the size of which is smaller than 50 kDa, with the exception of sulfated and non-sulfated polyanionic polysaccharides the size of which is greater than 50 kDa, for its use in the prevention and/or treatment of a bacterial infection.

2. Extract of algae for its use according to claim 1, wherein said polysaccharides comprise mannose and/or arabinose, in particular mannose.

3. Extract of algae for its use according to claim 2, wherein said polysaccharides comprise at least 0.005% mannose and/or at least 0.005% arabinose, by weight based on the weight of the total dry matter of the extract of algae.

4. Extract of algae for its use according to claim 3, wherein said polysaccharides comprise mannose in an amount ranging from 0.01 to 0.50% and/or arabinose in an amount ranging from 0.01 to 0.5%, by weight based on the weight of the total dry matter of the extract of algae.

5. Extract of algae for its use according to any one of claims 1 to 4, wherein said polysaccharides further comprise:
- galactose;
- glucose;
- rhamnose;
- xylose; and
- glucuronic acid.

6. Extract of algae for its use according to claim 5, wherein said polysaccharides comprise:
- from 0.05 to 0.5% galactose;
- from 0.005 to 0.5% glucose;
- from 2 to 15% rhamnose;
- from 0.1 to 1% xylose; and
- from 1 to 7% glucuronic acid;
by weight based on the weight of the total dry matter of the extract of algae.

7. Extract of algae for its use according to any one of the preceding claims, wherein the bacterial infection is an infection caused by a Gram-positive or Gram-negative bacterium.

8. Extract of algae for its use according to claim 7, wherein the bacterial infection is an infection caused by a Gram-positive bacterium chosen from *Staphylococcus aureus* and *Listeria monocytogenes* or a Gram-negative bacterium chosen from *Escherichia coli* and *Salmonella typhimurium.*

9. Extract of algae for its use according to claim 8, wherein the bacterial infection is an infection caused by *Staphylococcus aureus.*

10. Extract of algae for its use according to claim 9, wherein the bacterial infection is cutaneous.

11. Extract of algae for its use according to claim 7, wherein the bacterial infection is an infection caused by a bacterium chosen from *Pasteurella m. subsp septica, Pasteurella multocida subsp gallicida ST, Mannheimia haemolytica, Pasteurella multocida, Pasteurella multocida subsp multocida ST, Mannheimia, Erysipelothrix,* poultry *Staphylococcus aureus,* bovine *Staphylococcus aureus,* galline *Staphylococcus aureus* and *Streptococcus suis II.*

12. Extract of algae for its use according to any one of the preceding claims, wherein the extract of algae is comprised in a pharmaceutical composition.

13. Extract of algae for its use according to any one of claims 1 to 12, wherein the sulfated and non-sulfated polyanionic polysaccharides have a size smaller than or equal to 15 kDa.

14. Extract of algae for its use according to any one of claims 1 to 6, wherein said extract of algae is obtainable by a preparation method comprising the following steps:
a) the algae are washed and de-gritted;
b) said algae are ground;
c) the solid phase of the ground material is separated from its liquid phase;
d) said liquid phase is clarified;
e) the juice obtained in step d) is ultrafiltered through a membrane of less than 50 kDa; and
f) the filtered juice obtained in step e) is concentrated and then dried.

15. Extract of algae for its use according to claim 14, wherein the juice obtained in step d) of the preparation method is ultrafiltered through a membrane of 15 kDa or less.

16. Extract of algae for its use according to either claim 14 or claim 15, wherein the extract of algae obtained in step f) of the preparation method is purified, especially by ultrafiltration.
